# EUROPEAN PATENT APPLICATION

(11) **EP 0 561 119 A1**
(43) Date of publication of application: **22.09.1993**
(21) Application number: 93101054.0
(22) Date of filing: 25.01.1993
(51) Int. Cl.: A61L 27/00, A61K 35/36, A61K 47/42

(54) **Material of animal origin for use in surgery**

(30) Priority: 27.01.1992 IT RM920060
(71) Applicant: SPAZIO PROFESSIONALE S.r.l., I-82019 S.Agata dei Goti, Benevento (IT); Safoian, Asciot, I-81100 Caserta (IT); Cammarata, Armando, I-81100 Caserta (IT)
(72) Inventor: Safoian, Asciot, I-81100 Caserta (IT)
(74) Representative: Minoja, Fabrizio

(57) **Abstract**

A material of animal origin endowed with advantageous mechanical characteristics, a process for the preparation thereof, and methods of use thereof in surgery are herein described.

## Description

The present invention relates to a material of animal origin for use in surgery.

More particularly, the present invention relates to a material obtained from the skin of animals, such as bovines, equines and swine, to a method for the preparation thereof and to the use in plastic surgery.

Plastic surgery is going to find out new materials for implants.

Nowadays, the fibrillar proteins of connective tissue, particularly collagen, are used with good results. The implants made with collagen, which is used in the form of sponges or thin films, show several positive properties, but there are still several fields of plastic, ophthalmic and bone surgery that require more stringent properties to the materials to be used. Oculistic, bone and dental surgery are still in the need of a compatible, non antigenic material for implants, endowed with mechanic and tissue regenerative properties. Applications such as in the retina detachment, progressive myopia, bone and tissue regeneration in orthopedic and dental surgery are the critical fields wherein a fully compatible material is expected.

It has been found that processing the derm of animals, such as bovines, equines and swine, according to the process of the present invention, a new material, hereinafter named as product of animal origin responding to the above requirements is obtained.

The product of the invention is advantageously soft, elastic, and can be modelled into implants in the desired forms. Further the product of the invention can be easily sterilized with gamma rays without modifying the bio-mechanical characteristics.

The product of the present invention is absorbed in the implant site and the absorption time can properly be regulated. Another important advantage is the absence of anaphylactic reactions after the implant.

The product of animal origin can also be used as a carrier for active ingredients such as for example antibiotics, analgesics, antiinflammatories, vitamins, antihemorrhagics, etc.

A further object of the present invention is a process for the preparation of said product of animal origin. Basically, the process of the invention consists of the following steps:
a) cutting into pieces the previously washed and freezed skin of animals (such as for example cows, horses and pigs);
b) isolating the derm with usual techniques;
c) treating the so obtained derm fragments with a solution of 10% sodium hydroxide, borax and potassium hydrogenphosphate in bidistilled water at a temperature comprised between 3 and 5°C and adjusting pH at 8.65, for an adequate period of treatment, preferably 120 hours;
d) washing the product alternatively in distilled water and in alcohol-chloroform solution;
e) treating the product with a 80/20 sodium hydroxide/potassium dihydrogenphosphate solution at a temperature comprised between 18 and 20°C under stirring for two days;
f) treating the obtained material with pure sodium sulfate for 5 to 6 hours, then with boric acid till pH of 7.6 is reached;
g) subjecting the final product to crosslinking process by means of conventional crosslinking agents, such as formaldehyde vapors for a time depending on the desired absorption time in the implant site.
h) optionally shaping the final product in the form of tablets or films.

By a simple dehydration process the product of the invention can be obtained in the form of a powder. Also, the product of the present invention can be used in the form of a gel by suspending it in an appropriate liquid vehicle. The gel preparation is particularly advantageous in ophthalmic surgery because of its transparency.

The product of animal origin according to the present invention has been tested as to the toxicity on guinea pigs. Nor side effects nor anaphylaxis were observed.

The product of animal origin has been used in 150 patients affected by glaucoma, observing a fistulizing effect and the endoocular pressure stabilization. Clinical application of the product of the present invention in ophthalmology gave good results in the surgical treatment of retinopathies, in eyelid surgery, in the surgical treatment of myopia, corneal shock, intralamellar corneal partial transplant, as cornea shield, as substitute of vitreous humor in the form of gel, because of its transparency coefficient.

Another advantageous application of the product of the invention relates to dental surgery, when used as bone or gum regenerating agent.

The product of animal origin of the present invention can also be incorporated in pharmaceutical compositions in admixture with appropriate excipients. Said pharmaceutical compositions can be prepared according to conventional techniques, such as those described in "Remington's Pharmaceutical Science Handbook", XVII ed, Mack Pub.Inc.;USA. Examples of said pharmaceutical compositions are gels, ointments, creams, pellets, etc.

The following examples further illustrate the invention.

### EXAMPLE 1

1000 g of skin from big animals were washed with water and put in freezer for at least 12 hours, as to subsequently allow the longitudinal separation of the several layers and the isolation of the derm.

The derm is again put in freezer for at least 6 hours, and it is subsequently cut in pieces of the desired shape and size. Said pieces are put in a bidistilled water solution containing 10% sodium hydroxide, borax and potassium dihydrogen phosphate, at a temperature comprised between 3 and 5°C for 120 hours, keeping pH at the value of 8.65. The solution is renewed every 24 hours. After this time, the pieces are washed with distilled water for 3 hours and finally put in a alcohol-chloroform mixture in order to eliminate water. Then the pieces are kept in a 80-20 sodium hydroxidesodium sulfate at a temperature of 18-20°C under continuopus stirring for 2 days.

After that time, the pieces were immersed into pure sodium sulfate for 5-6 hours and in boric acid till neutralization of the solution (pH 7.6).

The so obtained pieces were exposed to formalin for a period of time ranging from 5 to 60 minutes according to the expected reabsorption time in the implant site.

About 1013 g of final product were obtained.

### EXAMPLE 2

450 g of the product obtained as in Example 1 were finely ground through a grinding apparatus having pore size from 6 down to 1.5 mm to obtain about 430 g of modellable paste.

### EXAMPLE 3

10 g of the product obtained as in Example 1 were suspended in 1 liter of a 5-10% Vitamin U sterile solution. The suspension was kept in a sterile chamber for 7-10 days at a temperature of 3-5°C.

### EXAMPLE 4

The product obtained as in Example 1 was essicated under vacuum to eliminate at least 80% humidity. The product was subsequently milled in a centrifugal mill and sieved through a 100-200 um sieve.

## Claims

1. A process for the preparation of a product of animal origin consisting of the following steps:
a) cutting into pieces the previously washed and freezed skin of animals selected from bovines, equines and swine,
b) isolating the derm with usual techniques;
c) treating the so obtained derm fragments with a solution of 10% sodium hydroxide, borax and potassium hydrogenphosphate in bidistilled water at a temperature comprised between 3 and 5°C and adjusting pH at 8.65, for an adequate period of treatment, preferably 120 hours;
d) washing the product alternatively in distilled water and in alcohol-chloroform solution;
e) treating the product with a 80/20 sodium hydroxide/potassium dihydrogenphosphate solution at a temperature comprised between 18 and 20°C under stirring for two days;
f) treating the obtained material with pure sodium sulfate for 5 to 6 hours, then with boric acid till pH of 7.6 is reached;
g) subjecting the final product to crosslinking process by means of conventional crosslinking agents.
h) optionally shaping the final product in the form of tablets or films.

2. A process according to claim 1 characterized in that the step g) is carried out in formaldehyde vapors for a time depending on the desired absorption time in the implant site.

3. A process according to claims 1 or 2 characterized in that the product obtained from step g) is dehydrated to obtain a powder.

4. A product of animal origin obtained from the process according to anyone of the claims 1 to 3.

5. The use of the product of claim 4 as implant material in surgery.

6. The use of the product of claim 4 as implant material in ophthalmic surgery.

7. The use of the product of claim 4 as implant material in dental surgery.

8. The use of the product of claim 4 as implant material in plastic surgery.

9. The use of the product of claim 4 as implant material in bone surgery.

10. Pharmaceutical compositions containing the product of claim 4 as active ingredient in the form of powder, tablets, films, gels, ointments, creams, pellets.

11. The use of the product of claim 4 as carrier for active ingredients, optionally in admixture with other conventional excipients in pharmaceutical compositions
